(19) Europäisches Patentamt
European Patent Office
Office européen des brevets

(11) Veröffentlichungsnummer: **0 150 350**
**B1**

(12) # EUROPÄISCHE PATENTSCHRIFT

(45) Veröffentlichungstag der Patentschrift: **25.01.89**

(51) Int. Cl.[4]: **C 08 F 218/08,** C 08 F 8/12

(21) Anmeldenummer: **84114854.7**

(22) Anmeldetag: **06.12.84**

(54) **Vernetzte Polymerisate, Verfahren zu ihrer Herstellung und ihre Anwendung.**

(30) Priorität: **13.12.83 DE 3344912**

(43) Veröffentlichungstag der Anmeldung: **07.08.85 Patentblatt 85/32**

(45) Bekanntmachung des Hinweises auf die Patenterteilung: **25.01.89 Patentblatt 89/4**

(84) Benannte Vertragsstaaten: **AT BE CH DE FR GB IT LI LU NL SE**

(56) Entgegenhaltungen:
**DE-A-1 444 831**
**FR-A-2 160 457**
**GB-A-1 304 189**
**US-A-3 082 194**

**CHEMICAL ABSTRACTS, Band 71, Nr. 6, September 1969, Seite 2, Nr. 50558z, Columbus, Ohio, US; S. MITSUHARU u.a.: "Copolymerization of allylidene diacetate with vinyl acetate"**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Mauz, Otto, Dr., Heidestrasse 21, D-6237 Liederbach (DE)**
Erfinder: **Noetzel, Siefried, Dr., An den Römergärten 3, D-6233 Kelkheim (Taunus) (DE)**
Erfinder: **Neumann, Bernhard, Dr., Stralsunder Strasse 15, D-3330 Helmstedt (DE)**

**EP 0 150 350 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Der Einsatz von Polymergelen zur Gelpermeationschromatographie von Polymerlösungen zum Zwecke der Stofftrennung bzw. Stoffreinigung oder zur Bestimmung der Molekulargewichtsverteilung ist bereits seit langem bekannt. Polymergele, die für wäßrige Systeme geeignet sind, werden als hydrophil bezeichnet, während solche, die nur in nichtwäßrigen Systemen (organ. Lösungsmitteln) Einsatz finden können, hydrophob genannt werden. Hydrophobe Gele sind beispielsweise vernetzte Polystyrole; hydrophile Gele sind demgegenüber solche auf Basis von vernetzten Dextranen, Polyvinylpyrrolidon, Polyacrylamid und Polyvinylalkohol. Einer zu starken Quellneigung derartiger Gele, die beispielsweise hohe Durchflußgeschwindigkeiten in der Gelpermeationschromatographie verhindert, kann durch die Erhöhung des Vernetzungsgrades entgegengewirkt werden.

Es sind schon hydrophobe Gele auf Basis von vernetztem Polyvinylacetat bekannt, aus denen durch Verseifung der Acetatgruppen ein hydrophiles Gel auf Basis von vernetztem Polyvinylalkohol hergestellt werden kann. Wichtig ist hier vor allem eine hohe Hydrolysebeständigkeit der Vernetzung.

Als Vernetzer sind für diesen Zweck bereits eine Reihe von Verbindungen im Stand der Technik beschrieben. So offenbart die DE-Patentschrift 1 517 935 hierfür neben Divinylalkylenen, Divinyl- und Dialkylestern von Dicarbonsäuren u.a. auch Divinyl- oder Diallylether von mehrwertigen Alkoholen. Bevorzugt ist dabei Butandioldivinylether (vgl. hierzu auch Makromol. Chemie 176, S. 657 ff (1975)). Die hiernach erhältlichen vernetzten Polyvinylacetate bzw. Polyvinylalkohole können auch in Form von makroporösen Perlen vorliegen. Die Vernetzung mit Butandioldivinylether ist zwar hydrolysestabil, jedoch copolymerisiert dieser Ether mit Vinylacetat nur relativ schwer, so daß sich damit nur relativ geringe Vernetzungsdichten erreichen lassen.

Andere bekannte Vernetzer wie Ethylenglykoldimethylacrylat oder Glycidylmethacrylat (US-Patentschrift 4 104 208) liefern keine hydrolysestabilen Vernetzungen und werden nicht gleichmäßig eingebaut. So liegen die Copolymerisationsparameter von Vinylacetat ($M_1$) und Metylmethacrylat ($M_2$) bei $r_1 = 0.01$ $r_2 = 20$. Ähnliche Copolymerisationsparameter sind für die hier genannten Methacrylate zu erwarten. Dies bedeutet, daß die beiden genannten Vernetzer zu Beginn der Reaktion verbraucht werden; ein gleichmäßiger Einbau ist daher nicht zu erzielen.

Es gehört gleichfalls schon zum Stand der Technik, hydrophile Polymergele für die Affinitätschromatographie zur Trennung biologisch aktiver Substanzen bzw. zur Immobilisierung derartiger Substanzen einzusetzen und dabei die reaktiven Gruppen des Polymergels, zumeist OH-Gruppen, zuvor mit sogenannten "Spacern" umzusetzen. Als Spacer kann dabei u.a. auch Epichlorhydrin dienen (vgl. DE-Offenlegungsschrift 2 102 514 und DE-Patentschrift 2 421 789).

Die Aufgabe vorliegender Erfindung bestand nun darin, ein vernetztes Polymeres auf Basis von Polyvinylester und insbesondere Polyvinylalkohol bereitzustellen, das die Nachteile des Standes der Technik nicht besitzt, das insbesondere hydrolysebeständig ist und sich als Adsorbens in der Gel-Chromatographie oder als Trägermaterial für chemisch kovalentgebundene, biologisch aktive Substanzen besonders gut eignet, die Aktivität der chemisch kovalent gebundenen, biologisch aktiven Substanzen wenig beeinträchtigt und einen leichten Durchfluß der zu behandelnden Substrate gewährleistet.

Die Erfindung betrifft daher ein vernetztes, perlformiges Polymerisat, das im wesentlichen aus Vinylacylat-Einheiten und Einheiten eines Vernetzungsmittels besteht, dadurch gekennzeichnet, daß das Vernetzungsmittel die allgemeinen Formeln

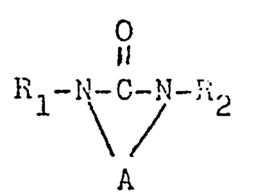

(I)

und/oder

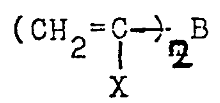

(II)

besitzt, wobei $R_1$, $R_2$ in Formel (I) gleich oder verschieden sein können und Vinyl-, 1-Acyloxy-Vinyl, Allyl- oder 2-Acyloxy-Allyl- bedeuten, A einen zweiwertigen Kohlenwasserstoffrest mit 2 bis 8 C-Atomen darstellt, B in Formel (II) für einen zweiwertigen Kohlenwasserstoffrest mit 1 bis 8 C-Atomen steht, und X Acyloxy-bedeutet, und wobei die Menge an Vernetzungsmittel-Einheiten 0,1 bis 60 Gew.-%, bezogen auf das Polymere, beträgt, wobei die Acyloxy-Gruppen der Vinylacylat-Einheiten als solche vorliegen oder teilweise oder vollständig durch Hydroxylgruppen ersetzt zind, und wobei die mittlere Teilchengröße der Perlem im trockenel, ungequollenen Zustand 20 bis 800 µm und der mittlere Porendurchmesser 2 bis 10000 nm beträgt. Bevorzugt

sind die Acylatgruppen der Vinylacylat-Einheten teilweise oder vollständig durch OH-Gruppen ersetzt.

Die Erfindung hat weiterhin ein Verfahren zur Herstellung eines derartigen vernetzen Polymerisates zum Gegenstand durch Copolymerisation von Vinylacylat mit einem Vernetzungsmittel in Gegenwart eines Dispersionsmittels, dadurch gekennzeichnet, daß das Vernetzungsmittel die obigen Formeln (I) und/oder (II) besitzt. Bevorzugt wird das so erhaltene Polymerisat anschließend pertiell oder vollständig verseift.

Schließlich bezieht sich die Erfindung auch auf die Verwendung der erfindungsgemäßen Polymerisate als Adsorbens in der Chromatographie oder als Trägermaterial für biologisch aktive Substanzen.

Die Vinylacylat-Einheiten des erfindungsgemäßen Polymerisates enthalten vorzugsweise 2 bis 18 C-Atome, insbesondere 2 bis 6 C-Atome im Acylatrest. Bevorzugt ist dies der Acetat- oder Propionatrest. Es können auch verschiedene Acylatreste im Polymerisat vorhanden sein, d.h. zu seiner Herstellung können auch Gemische der entsprechenden Vinylacylate eingesetzt werden.

In dem Vernetzungsmittel gemäß der Formel (I) stellt A bevorzugt einen verzweigten oder unverzweigten aliphatischen Kohlenwasserstoffrest mit 2 bis 5 C-Atomen, insbesondere 2 oder 3 C-Atomen dar. Besonders bevorzugt ist dies der Ethylen- oder Propylenrest. Falls $R_1/R_2$ dieser Formel (I) für 1-Acyloxy-Vinyl- oder 2-Acyloxy-Allyl-stehen, so enthält die Acyloxy-Gruppe darin vorzugsweise 2 bis 18 C-Atome, insbesondere 2 bis 6 C-Atome. Bevorzugt bedeutet Acyloxy- den Acetat- oder Propionatrest. Vorzugsweise haben die Reste $R_1/R_2$ die Bedutung von Vinyl-. Eine bevorzugte Vernetzer-Einheit in dem erfindungsgemäßen Polymerisat leitet sich dementsprechend von N,N'-Divintyl-ethylenharnstoff ab. Dieser Vernetzer bewirkt eine besonders hydrolysebeständige Verknüpfung. Ein weiterer bevorzugter Vertreter ist N,N'-Divinylpropylenharnstoff.

Die Herstellung derartiger Verbindungen ist bekannt und beispielsweise beschrieben in der US-Patentschrift 2 541 152 oder in Ullmann, Encyklopädie der technischen Chemie, Bd. 23, 611 (4. Auflage).

In dem Vernetzungsmittel gemäß der Formel (II) hat B bevorzugt die Bedeutung eines zweiwertigen Kohlenwasserstoffrestes, insbesondere eines veriweigten oder unverzweigten Alkylenrestes mit 2 bis 6 C-Atomen, vorzugsweise 2 bis 4 C-Atomen. Die Acyloxy-Gruppe hat hier vorzugsweise die gleiche Bedeuteng wie oben für den Rest R in der Formel (I) beschrieben. Ein bevorzugter Vernetzer dieser Art ist beispielsweise 3,3-Dimethylpentadien-2,4-diacetat, das besonders leicht mit dem Vinylacylat copolymerisiert. Die Herstellung derartiger Verbindungen kann beispielsweise durch Umsatz des entsprechenden Di-, Tri- oder Tetraketons mit Vinylacylat oder Isopropenylacylat in Gegenwart saurer Katalysatoren unter Bildung der entsprechenden Enolacylate erfolgen. Das gleichzeitig enstehende Aceton muß dabei laufend durch Destillation aus dem Gleichgewicht entfernt werden.

Die Menge an Einheiten des Vernetzungsmittels (II) beträgt im allgemeinen 0 bis 100 %, insbesondere 0 bis 60 %, bezogen auf die Gesamtmenge an Vernetzer-Einheiten im Polymerisat.

Die Gesamtmenge an Vernetzer-Einheiten in dem erfindungsgemäßen Polymerisat liegt in den beanspruchten Bereichen und hängt von der für den jeweiligen Anwendungszweck gewünschten Vernetzungsdichte ab. So wird beispielsweise bei der Gelchromatographie eine hohe Formstabilität angestrebt, was eine hohe Vernetzungsdichte und damit einen höheren Gehalt an vernetzenden Monomer-Einheiten zur Voraussetzung hat. Demgegenüber kann in anderen Anwendungsbereichen, beispielsweise als Trägermatreial für Enzymereaktionen im Rührkessel oder für Diagnostika eine geringere Vernetzerdichte vorteilhaft sein. Vernetzergehalte unterhalb von 0,1 Gew.-% führen in den meisten Fällen zu nicht mehr brauchbaren Produkten. Vernetzergehalte oberhalb von 60 Gew.-% sind grundsätzlich möglich; erbringen in der Regel jedoch keine weiteren Vorteile.

Je nach Anwendungszweck liegt die Menge an Vernetzer-Einheiten vorzugsweise bei 1 bis 50 Gew.-%, und insbesondere bei 1 bis 40 Gew.-%, bezogen auf das Polymere. Bei Einsatz als Trägermaterial für biologisch aktive Substanzen liegt die Untergrenze bevorzugt bei 2,5 Gew.-% und besonders bevorzugt bei 10 Gew.-%. Falls nur Vernetzer-Einheiten gemäß Formel (II) vorliegen, so beträgt deren Untergrenze besonders bevorzugt 2,5 Gew.-%.

Für manche Anwendungszwecke kann es von Vorteil sein, wenn das erfindungsgemäße Polymerisat zusätzlich noch Monomer-Einheiten eines mit Vinylacylat copolymerisierbaren Monomeren enthält, wobei deren Menge im allgemeinen 10 Gew.-%, bezogen auf das Gesamtpolymere, nicht überschreitet und vorzugsweise zwischen 0,1 und 5 Gew.-% liegt. Beispiele für derartige Monomere, die gegebenenfalls im Gemisch eingesetzt werden können, sind: N-Vinylpyrrolidon, Vinylencarbonat, (Meth)acrylsäure, (Meth)acrylnitril, (Meth)acrylamid, (Meth)acrylsäurealkylester mit jeweils 2 bis 12 C-Atomen, vorzugsweise 2 bis 4 C-Atomen im Alkylrest, Hydroxyalkylester der (Meth)acrylsäure mit 2 bis 6 C-Atomen in der Alkylgruppe, N-Vinyl-N-alkylacetamid, Styrol, α-Methylstyrol udgl.

Das erfindungsgemäße vernetzte Polymerisat liegt in Form von Perlen vor, die überwiegend kugelförmige Gestalt aufweisen, deren mittlere Teilchengröße im trockenen, ungequollenen Zustand 20 bis 800 µm, vorzugsweise 50 bis 300 µm beträgt und die vorzugweise eine enge Teilchengrößenverteilung aufweisen. Das jeweilige Optimum der Teilchengröße hängt dabei vor allem von dem speziellen Einsatzgebiet ab. Bei einem ohne Druck durchgeführten Säulenverfahren wird man beispielweise die Teilchengröße innerhalb der vorstehend genannten Grenzen entsprechend größer wählen als bei einem Druck-Verfahren. Die Perlen des erfindungsgemäßen Polymerisates sind überwiegend makroporös ausgebildet. Der mittlere Porendurchmesser liegt im Bereich von 2 bis 10000 nm, vorzugsweise 5 bis 200 nm und insbesondere 20 bis 200 nm.

Die Bestimmung des Porendurchmessers (Porenvolumens) kann in der Weise erfolgen, daß zunächst das Porenvolumen gemäß der Kapillardruckmethode (Quecksilberporosimetrie) bestimmt wird (vgl. hierzu "Ullmanns Encyklopädie der technischen Chemie", Bd. 5 (1980), S. 751 - 752). Daraus ergibt sich dann der

mittlere Porendurchmesser durch Berechnung nach der auf Seite 752, linke Spalte oben, dieser Literaturstellen angegebenen Gleichung. Daneben ist eine Porengrößenbestimmung auch durch Rasterelektronenmikroskopie möglich.

Die Acylat-Gruppen der Vinylacylat-Einheiten im erfindungsgemäßen Polymerisat sind vorzugseise zu OH-Gruppen verseift, wobei der Verseifungsgrad z. B. mindestens 10 %, im allgemeinen mehr als 50 %, vorzugsweise mehr als 70 % und insbesondere 90 bis 100 % beträgt. Bei Einsatz als Trägermaterial für biologisch aktive Substanzen ist in dem durch Verseifung erhaltenen vernetzten Polymeren (Polyvinylalkohol) vorzugsweise zumindest ein Teil der OH-Gruppen durch sogenannte "Spacer"-Gruppen besetzt (bezüglich "Spacer" siehe weiter unten). Für manchc Zwecke der Gelchromatographie kann es demgegenüber von Vorteil sein, zumindest einen Teilen der OH-Gruppen mit hydrophobierenden Gruppen, die keine reaktiven Reste mehr enthalten, zu belegen.

Die erfindungsgemäßen Polymerisate zeichnen sich insbesondere durch eine hohe Hydrolysenbeständigkeit bei hoher Vernetzungsdichte aus. Diese hohe Hydrolysenbeständigkeit ist nicht nur in der Gelchromatographie, sondern auch bei Einsatz als Trägermaterial für biologisch aktive Substanzen, wie Enzyme von großer Bedeutung. Auf Träger fixierte Enzyme werden häufig über Jahre im stark alkalischen oder stark sauren Milieu eingesetzt. Dies trifft im besonderen Maße für die "unspezifischen Hydrolasen" zu, die Ester- oder Carbonsäureamid-Bindungen spalten. Im übrigen ist die stabile Vernetzung auch bei der Verseifung der Acylat-Gruppen zu OH-Gruppen in den erfindungsgemäßen Polymerisaten vorteilhaft.

Die erfindungsgemäßen Polymerisate eignen sich u.a. als stationäre Phase in der Gelchromatographie und als Trägermaterial für biologisch aktive Substanzen.

Die Herstellung des erfindungsgemäßen vernetzten Polymerisates erfolgt in bekannter Weise, vorzugsweise unter den Bedingungen der Perlpolymerisation, in Gegenwart eines Dispersionsmittels, eines Dispersionsstabilisators und gegebenenfalls weiterer Zusatzstoffe sowie gegebenenfalls eines radikalisch wirksamen Initiators und vorzugsweise eines inerten Verdünnungsmittels sowie unter Ausschluß von Sauerstoff.

Als Dispersionsmittel zur Durchführung der Perlpolymerisation dienen vor allem solche Verbindungen, die unter Normalbedingungen flüssig sind, einen Siedepunkt von oberhalb 60°C, vorzugsweise im Bereich von 85 - 300°C, aufweisen und welche die Monomeren, das Polymere und vorzugsweise auch den Initiator unter den Polymerisationsbedingungen nicht oder jedenfalls nur spurenweise lösen, um eine Emulsionspolymerisation zu unterbinden. Das Verhältnis der Monomerphase zur Dispersionsmittelphase kann in weiten Grenzen variieren, beispielsweise zwischen 0,5 : 1 bis 1 : 50, vorzugsweise 1 : 1 bis 1 : 15 (Gewichtsverhältnis). Bevorzugt dient erfindungsgemäß als Dispersionsmittel Wasser. Vorteilhafterweise enthält dieses einen Puffer, der im alkalischen Bereich arbeitet und der durch Hydrolyse von Vinylacylat gebildete Säure abfängt. Dieser Puffer besteht vorzugsweise aus $Na_2HPO_4$/ $NaH_2PO_4$ bzw. aus $NaHCo_3$.

Als Dispersionsstabilisator, der ein Agglomerieren der Perlen während der Polymerisation verhindern soll, dienen die hierfür bekannten Verbindungen. Vorzugsweise ist diese ein hydrophiles Polymer wie Polyvinylpyrrolidon, Polyvinylalkohol, Polyacrylamid, Polyethylenglykol, Methylcellulose oder Ethylenoxid -Propylenoxid-Copolymere.

Polyvinylpyrrolidon wird für diesen Zweck besonders bevorzugt. Diese Dispersionsstabilisatoren sind bereits in Mengen von 0,001 Gew.-%,, bezogen auf die Gesamtmenge an Monomeren, wirksam. Zumeist werden Mengen von 0,005 bis 50 Gew.-%, vorzugsweise 0,01 - 20 Gew.-% (bezogen auf die Gesamtmenge an Monomeren) verwendet.

Der Zusatz eines Elektrolyten (im Falle von Wasser als Dispersionsmittel), beispielsweise eines Salzes wie Kochsalz zur wäßrigen Phase ist im allgemeinen vorteilhaft, da er die fast vollständige Verdrängung der Monomeren aus der äußeren Phase, dadurch eine fast völlige Unterdrückung von Emulsionsbildung und daneben eine Steigerung der Perlausbeute bewirkt. Der Elektrolytzusatz kann darüber hinaus teilweise auch die Wirkung eines Schutzkolloids haben. Zumeist wird dieser Elektrolyt in Mengen bis zu 50 Gew.-%, vorzugsweise bis zu 30 Gew.-%, bezogen auf das Dispergiermittel, verwendet.

Als radikalisch wirksame Initiatoren kommen erfindungsgemäß solche in Betracht, die in der Monomerphase guter und in dem flüssigen Dispergiermittel möglichst wenig löslich sind. Beispiele hierfür sind organische Peroxide, wie Di-tert.-butylperoxid, Dibenzoylperoxid, Cumolhydroperoxid, Cyclohexanonperoxid oder aliphatische Azoverbindungen, wie α,α'-Azodiisobuttersäurenitril, Azo-bis-cyanvaleriansäure, 1,1'-Azo-cyclohexan-1,1'-dicarbonsäuredinitril und Azodicarbonamid. Gegebenenfalls können auch entsprechende Redoxsysteme Verwendung finden. Die Menge an Initiator beträgt zumeist 0,01 - 5 Gew.-%, vorzugweise 0,1 bis 2 Gew.-% (bezogen auf die Gesamtmenge der Monomeren). Möglich ist auch die Initiierung der Polymerisation durch Strahlung, ggf. bei gleichzeitiger Anwesenbeit eines Initiators.

Um eine möglichst hohe Porosität der Perlpolymerisate zu erreichen, werden dem Polymerisationssystem oder vorzugsweise den Monomeren bestimmte inerte, flüssige Komponenten zugesetzt (Verdünnungsmittel). Hierunter sollen solche Stoffe verstanden sein, in denen sich die Monomeren gut lösen oder mit ihnen mischbar sind, andererseits aber im Dispersionsmittel praktisch unlöslich und damit mit diesem nicht mischbar sind. Derartige Verdünnungsmittel und ihre Wirkungsweise sind beispielsweise beschrieben in der DE-Patentschrift 1 517 935 sowie in Makromol. Chemie 176, S. 657 ff (1975).

Das optimale Verdünnungsmittel bzw. Verdünnungsmittelgemisch läßt sich durch einige einfache Routineversuche leicht ermitteln. Die Porengröße ist durch Art und Zusammensetzung sowie der Menge der Inertkomponente beeinflußbar, hängt aber auch von der Menge an vernetzender Komponente ab.

Die Verdünnungsmittel können allein oder in Mischung eingesetzt werden und Lösungs- oder Fällungsmittel für Polyvinylacetat sein. Als Beispiele seien genannt: Alkanole wie Butanol, Cyclohexanol, Isooctanol, Glykol, oder Ester wie Butylacetat, Butylglykolacetat, Glycerintriacetat, oder Amide wie Dimethylformamid, Dimethylacetamid, Pyrrolidon oder Ketone wie Aceton, Cyclohexanon oder Ether, Dialkylether mit mindestens 6 C-Atomen wie Di-n-butylether, Di-n-amylether, Diphenylether oder Kohlenwasserstoffe wie Hexan, Benzol, Isooctan, Paraffinöl. Bevorzugt werden - im Falle von Wasser als Dispersionsmittel - als Verdünnungsmittel, Dialkyläther mit mindestens 6 C-Atomen wie Di-n-butyläther oder Di-n-amyläther eingesetzt. Weitere bevorzugte Verdünnungsmittel sind Polyglykole, die durch Anlagerung eines Gemisches von Äthylenoxyd und Propylenoxyd oder von Propylenoxyd allein an einem Alkohol, z. B. Butanol als Startmolekül entstehen, und die gegebenenfalls eine statische Äthylenoxyd-Propylenoxydverteilung aufweisen oder Blockpolymere aus Äthylenoxid und Propylenoxid, bei denen Poly(oxyethylen)-Einheiten an beiden Enden der Poly(oxypropylen)-Kette addiert sind.

Die Menge an zugesetztem Verdünnungsmittel ist weitgehend variabel. Sie hängt u.a. von der Monomerzusammensetzung, insbesondere des Gehaltes an Vernetzer, der erwünschten Porosität (Porengröße) sowie vom genauen Verwendungszweck des Polymeren ab. So wird sich bei einem hohen Vernetzungsgrad eine entsprechend große Menge ab Verdünnungsmittel empfehlen, um eine bestimmte Porosität (Porengröße) zu erreichen. Bei ein und demselben Vernetzungsgrad wird die Porosität (Porengröße) gleichfalls umso größer sein, je mehr an Verdünnungsmittel eingesetzt wird. Naturgemäß läßt sich dies nur innerhalb bestimmter Grenzen steigern, da ansonsten die mechanischen Festigkeit zu gering wird. In den meisten Fällen wird ein Volumen an Verdünnungsmittel, das dem 0,02- bis 5-fachen, vorzugsweise dem 0,04- bis 3-fachen Volumen an eingesetzten Monomeren entspricht, zufreidenstellende Ergebnisse liefern.

Das Vinylacylat sowie der Vernetzer und das weitere (bzw. die weiteren Comonomere(n) werden in solchen Menge eingesetzt, daß ein Polymeres mit den weiter oben angegebenen mengen an Monomer-Einheiten resultiert.

Das erfindungsgemäße Verfahren wird zweckmäßigerweise in einem mit einer Rührvorrichtung versehenen Reaktionsgefäß bei Temperaturen von zumeist 20 - 150° C, vorzugsweise 20 - 100° C und einem Druck von 1 - 10 bar, vorzugsweise 1 - 5 bar durchgeführt. Die Teilchengröße des Perlpolymerisates wird in bekannter Weise durch die Rührgeschwindigkeit und das Phasenverhältnis eingestellt. Das Reaktionsgefäß ist vorzugsweise vakuumfest und kann mit Rückflußkühler, Zulauftrichter, Gaseinleitungsrohr und Temperaturmeßgerät versehen werden. Die Beheizung und Kühlung des Gefäßes erfolgt im allgemeinen durch ein Flüssigkeitsbad, z. B. ein Ölbad oder Wasserbad. Es ist vorteilhaft, das erfindungsgemäße Verfahren unter Ausschluß von Luftsauerstoff durchzuführen. Das Reaktionsgefäß wird daher vor Beginn mit einem interten Gas, vorzugsweise Stickstoff, gespült.

Nach Beendigung der Polymerisationsreaktion werden die nicht umgesetzten Monomeren aus dem Reaktionsgefäß entfernt, z. B. durch Verdampfen bei vermindertem Druck, vorzugsweise bei einem Druck von 0,1 - 15 Torr. Nach der Entfernung der Restmonomeren wird das Dispersionsmittel vom festen Polymeren abgetrennt, z. B. durch Dekantieren, Filtrieren oder Absaugen des Überstandes. Das gegebenenfalls eingesetzte Verdünnungsmittel kann zuvor durch Wasserdampfdestillation entfernt werden. Anschließend wird das Polymerisat, falls erforderlich, mit leichtsiedenden organischen Lösungsmitteln, z. B. einem Kohlenwasserstoff, einem niederen Alkohol oder Aceton gewaschen und schließlich getrocknet. Die Trocknung des Polymerisates erfolgt bei einer Temperatur von zumeist 200 - 100° C, vorzugsweise von 20 - 80° C; eine Trocknung unter vermindertem Druck ist dabei empfehlenswert.

Das so erhaltene Polyvinylacylatgel ist nicht hydrophil; zur Anwendung in Wasser muß die Estergruppe hydrolysiert werden. Das kann in bekannter Weise alkalisch durch Quellen des Produktes in einem Alkohol wie z. B. Methanol und Zugabe von wäßrigem Alkali wie z. B. Natronlauge geschehen oder durch Umesterung des alkoholgequollenen Produkts mit katalytischen Mengen Säure oder Base bei laufender z. B. destillativer Entfernung des gebildeten Esters (vgl. DE-Patentschrift 1 517 935). Die Verseifung kann auf jeder beliebigen Stufe abgebrochen werden, so daß je nach Verwendungszweck der Grad der Hydrophilie des dels eingestellt werden kann.

Falls das perlförmige vernetzte Polyvinylalkoholgel als Träger für biologisch aktive Substanzen, die durch eine kovalente Bindung an den Träger fixiert werden sollen, eingesetzt wird, so ist es in vielen Fällen zweckmäßig, das Gel zuvor mit sogenannten " Spacern" zu modifizieren. Unter "Spacer" versteht man dabei Verbindungen die sowohl mit dem Trägerpolymeren als auch mit der biologisch aktiven Substanz reagieren und zwischen beiden gewissermaßen eine Brücke bilden. Die Umsetzung des Perlpolymerisates mit dem Spacer kann entweder direkt oder vorzugsweise nach vorheriger Verseifung der Acylatgruppen erfolgt. Der Umsatzgrad hängt dabei u.a. von der Sperrigkeit des Spacers und der Zugänglichkeit der Acylatgruppe bzw. der daraus entstandenen sekundären Hydroxylgruppen ab. Als Spacer kommen erfindungsgemäß die hierfür bekannten homo- und hetero-bifunktionellen Verbindungen in Frage, deren zweite funktionelle Gruppe die Kopplung mit der zu fixierenden biologisch aktiven Substanz übernimmt (vgl. die DE-Patentschrift 2 421 789 und 2 552 510, sowie Ullmans Encyclopädie der technischen Chemie, 4. Auflage, Bd. 10, S. 540 und "Characterization of Immobilized Biocatalysts", Verlag Chemie, Weinheim, 1979, S. 53).

Bei den erfindungsgemäß eingesetzten Spacern handelt es sich beispielsweise um solche, welche die nachstehenden Gruppen einführen:

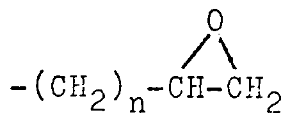 n= 1 - 8

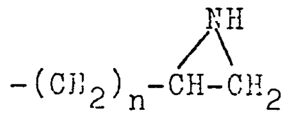 n= 1 - 8

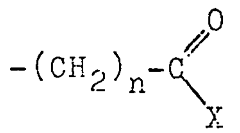 ; n= 1 - 8
x= H, OH, Halogen, $N_3$, OR

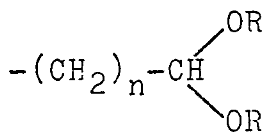 ; n= 1 - 6
R= Alkylrest mit 1 - 6 C-Atomen

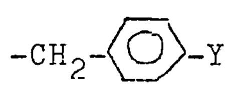 ; Y= $NH_2$, $N_2$, NCO.

Bevorzugte "Spacer" sind erfindungsgemäß solche, die hydrolysebeständige chemische Bindungen herbeiführen, wie Epichlorhydrin oder dessen Homologe (α,β-Expoxy-ω-halogenalkane). Die Umsetzung der Polyvinylalkohole (Polyvinylacylate) erfolgt dabei ohne Lösungsmittel oder in Gegenwart eines Lösungsmittels, vorzugsweise in Anwesenheit eines Katalysators. Die Umsetzungsdauer liegt - abhängig von der Temperatur - die zwischen Raumtemperatur und Rückflußtemperatur des Epichlorhydrins (113 - 115°C) liegen kann - im allgemeinen zwischen 30 Minuten und 24 Stunden. Als Katalysatoren kommen z. B. NaOH (in Pulverform) oder wäßrige Alkalien, Dimethylformamid, Triethylamin und andere Säureakzeptoren in Frage.

Unter dem Begriff biologisch aktive Substanzen seien die bekannten in vivo oder in vitro wirksamen natürlichen oder künstlich hergestellten Stoffe verstanden, wie Enzyme, Aktivatoren, Inhibitoren, Antigene, Antikörper, Vitamine, Hormone, Effektoren, Antibiotika, Proteine udgl. Der letztere Begriff umfaßt dabei auch Proteine mit bestimmten Nicht-Proteinsubstituenten wie Metallionen, Polysacchariden, Porphyringruppen, Adenindinucleotid, Ribonucleinsäure, Phospholipide etc. Auch Polypeptidfragmente, z. B. die aktiven Teile von Enzymmolekülen, fallen unter den Begriff biologisch aktive Substanzen.

Von den vorstehend genannten biologisch aktiven Substanzen sind erfindungsgemäß die Enzyme bevorzugt. Beispiele für Enzyme sind Urease, Penicillinacylase, D-Aminosäureoxidase, Adenyldesaminase, Alkohol-Dehydrogenase, Asparaginase, Carboxypeptidase, Chymotrypsin, Diphosphoesterase, α-Glucosidase, Glucose-Isomerase, Glucose-Oxidase, Glucose-6-phosphat-Dehydrogenase, Hexokinase, Invertase, β-Lactamase, Lactase, Lactat-Dehydrogenase, versch. Lectine, NAD-Kinase, Neuraminidase, Papain, Peroxidase, Phosphatasen (alkalisch und sauer), 5'-Phosphodiesterase, Pyruvat Kinase, Ribonuclease, Trypsin.

Beispiele für andere biologisch aktive Substanzen sind Hormone, wie Insulin und die verschiedensten Hypophysen-Hormone, Proteine der gamma-Globulinfraktion, z. B. Antikörper der Klasse G, M, A, D und E, andere Blutfaktoren, z. B. Antihämophiliefaktor, die Blutgerinnungsfaktoren, spezielle Antikörper, z. B. Hepatits-, Poliomyelitis-, Finnen-, Mumps-, Influenza- oder Kaninchenantikörper, Antigene, wie Hepatits-, Polymyelitis-, Finnen-, Mumps-, Influenza- oder Kaninchenantigene zur Reinigung oder Stimulierung geeigneter Antikörperreaktionen, wobei das Antigen (nach dem Unlöslichmachen) in der unlöslichen Form verbleibt und folglich nicht in den Körper eindringen und diesen schädigen kann, so wie allgemeine Körperproteine, wie Hämoglobin oder Albumin.

Die Verankerungsreaktion zwischen der biologisch aktiven Substanz wird in bekannter Weise durchgeführt, wie etwa in der DE-OS 2 407 340 oder in den DE-PSen 2 215 687, 2 421 789 und 2 552 510 beschrieben. Zumeist erfolgt die Umsetzung bei Raumtemperatur bzw. bei +40°C oder darunter liegenden Temperaturen. Letzteres insbesondere dann, wenn die zu verankernde biologisch aktive Substanz von Hause aus instabil ist; in diesem Fall liegen dann die Temperaturen unterhalb von +10°C, vorzugsweise bei 0 bis +5°C.

Die Verankerungsreaktion erfolgt vorzugsweise in der Umgebung eines neutralen pH-Wertes, beispielsweise bei pH-Werten von 5 - 9, da hier die meisten biologisch aktiven Substanzen am stabilsten sind. In der Regel ist es auch nicht erforderlich, stärker saure oder alkalische Bedingungen einzuhalten, da die makroporösen

Perlpolymerisate auch bereits im Neutralbereich mit den meisten der in Frage kommenden Substanzen schnell reagieren. Die dabei entstehende Bindung bietet genügend Stabilität für lange Lagerungen und hohe Operationsstabilität.

Die Erfindung wird nachfolgend anhand von Beispielen näher erläutert.

**Beispiel 1**

In einem Kolben mit Rührer, Thermometer und Rückflußkühler wurde unter Stickstoffatmosphäre eine organische Phase, bestehend aus einer Lösung von 97,5 g Vinylacetat, 2,5 g Divinylethylenharnstoff und 0,1 g Azoisobutyronitril unter Rüsren in einer wäßrigen Phase, bestehend aus 4,2 g $Na_2HPO_4$, 0,25 g $NaH_2PO_4$, 7,0 g Polyvinylpyrrolidon und 700 ml $H_2O$ suspendiert. Durch Erwärmen auf 75°C mittels eines Heizbades wurde die Polymerisation gestartet. Nach zwei Stunden wurde die Temperatur auf 85°C erhöht. Weitere zwei Stunden später war die Polymerisation beendet. Die erhaltene Suspension wurde durch Eingießen in Eis abgekühlt, nach mehrmaligem Abdekantieren der feinteiligen Emulsion das Polymerisat abfiltriert und getrocknet. Es wurden 80 g trockenes Produkt erhalten.

Zur Hydrolyse wurden 50 g des Produktes in Methanol gequollen und mit einer Lösung von 50 g NaOH in $H_2O$ bei 25°C ohne Erwärmung oder Gegenkühlung versetzt. Nach zwölf Stunden filtrierte man das Produkt ab, wusch mit viel Wasser bis zur Neutralität und trocknete.

Die Produkte wurden in der Gelchromatographie eingesetzt. Für das unverseifte Gel wurde in Tetrahydrofuran mit Polystyrol ein Ausschlußmoklekulargewicht von 1200 gefunden. Das verseifte Produkt ergab für Polyethylenglykol in Wasser ein Ausschlußmolekulargewicht von 1100.

**Beispiel 2**

Die Polymerisation des Beispiels 1 wurde unter Zusatz von 140 g NaCl zur wäßrigen Phase durchgeführt. Nach Beendigung der Reaktion durch Eingießen in Eiswasser setzte sich sofort alles Perlpolymerisat am Boden ab, so daß fast kein Emulsionsanteil erhalten wurde. Die isolierbare Ausbeute an Perlpolymerisat lag über 90 % (bezogen auf die polymerisierbare Phase).

Das Ausschlußmolekulargewicht des Gels für Polystyrol in Tetrahydrofuran stieg auf 1500; nach der Verseifung erhielt man in Wasser für Polyethylenglykol ein Ausschlußmolekulargewicht von 1300.

**Beispiel 3**

Die Polymerisation von Beispiel 1 wurde analog durchgeführt mit der Ausnahme, daß die Vernetzungskomponente aus 2,0 g Divinylethylenharnstoff und 0,5 g 3,3-Dimethyl-pentadien-2,4-diacetat bestand.

Die gelchromatographischen Daten entsprachenenen des Beispiels 1.

Die Herstellung des 3,3-Dimethylpentadien-2,4-diacetats erfolgte, wie nachstehend beschrieben, durch Acylierung von 3-Methyl-butanon-(2) mit Acetanhydrid in Gegenwart einer Lewis-Säure und anschließendem Umsatz des so erhaltenen Diketons mit Isopropenylacetat:

330 g (3,84 Mol) frisch destilliertes 3-Methylen-butanon wurden mit 500 g (5 Mol) technischem Acetanhydrid (95 %) in einem Kolben gemischt und unter Rühren und Überleitung von Stickstoff 160 g (1,15 Mol) $ZnCl_2$ zugeben. Der Kolbeninhalt wurde für 3 Stunden auf 120°C erhitzt und nach dem Abkühlen im Wasserstrahlvakuum destilliert.

Es wurden 363 g eines Rohproduktes mit einem Siedepunkt von 62°C (12 torr) in einer GC-Reinheit von 86 % erhalten.

Die erneute Destillation dieser Substanz im Wasserstrahlvakuum ergab 289 g eines einheitlichen Produkts mit Siedepunkt 60°C (12 torr), das sich durch $^1$H-NMR-Analyse als 2,2-Dimethyl-pentan-2,4-dion erwies (Singulett 1,3 ppm und 2,05 ppm).

110 g 3,3-Dimethyl-pentan-2,4-dion wurden unter Stickstoff mit 500 g trockenem Isopropenylacetats gemischt und 5 g p-Toluol-sulfonsäure zugefügt. Das Gemisch wurde an einer kurzen Füllkörperkolonne zum Rückfluß erhitzt und in Zeitabständen von 12 Stunden über mehrere Tage jeweils kurzzeitig Destillatfraktionen zwischen 54°C und 90°C abgenommen. Die Zusammensetzung des Reaktionsgemischs wurde gasehromatographisch verfolgt. Nach Ablauf der Reaktionszeit von 6 Tagen wurde der saure Katalysator durch Carbonatzugabe neutralisiert und das Reaktionsgemisch im Wasserstrahlvakuum schnell abdestilliert. Durch erneute Destillation bei Normaldruck wurde nicht reagiertes Isopropenylacetat entfernt. Durch Anlegen von Wasserstrahlvakuum wurden bei erneuter Destillation 39 g (87,5°C/ 12 torr) einer gaschromatographisch reinen Substanz erhalten. Bei diesem Produkt handelte es sich um 3,3-Di-methyl-pent-1-en-4-on-2-acetat ($^1$H-NMR:Singulett 1,3 ppm und 2,1 ppm Duplett 4,9 ppm).

Die weitere Destillation des restlichen Rohprodukts im Ölpumpenvakuum bei 0.01 torr führt zu einem Produktgemisch aus dem Monoenolacetat und einer zweiten, höher siedenden Verbindung. Daraus wurden zwischen 54°C und 65°C bei 0.01 torr zusätzlich 15 g des Dienolacetats in einer Reinheit von 93 % (GC) erhalten.

**Beispiel 4**

Zur Durchführung einer heterogen-vernetzenden Perlcopolymerisation wurde eine Lösung von 80 g Vinylacetat, 20 g Divinylethylenharnstoff, 1 g Azoisobutyronitril und 200 g n-Heptanol in der Lösung von 0,175 g $NaH_2PO_4$, 3 g $Na_2HPO_4$ und 5 g Polyvinylpyrrolidon in 500 ml Wasser dispergiert und polymerisiert. Der Temperaturverlauf entsprach dem in Beispiel 1. Nach vier Stunden wurde das Verdünnungsmittel durch Wasserdampfdestillation entfernt und das Produkt isoliert. Die Ausbeute betrug 77,7 g an völlig rundem klarem Perlpolymerisat. Der mittlere Partikeldurchmesser lag bei etwa 30 µm (Rührgeschwindigkeit von 450 upm).

Das Produkt hatte ein Schüttvolumen von 1,55 ml/g. In Tetrahydrofuran betrug sein Gelbettvolumen 5,77 ml/g, das Ausschlußmolekulargewicht für Polystyrol lag bei 80.00. Das verseifte Produkt hatte ein Schüttvolumen von 1,54 ml/g, es quoll in Wasser auf 5 ml/g und zeigte ein Ausschlußmolekulargewicht für Polyethylenglykol von 20.000.

20 g des hydrolysierten Perlcopolymerisates ließ man in 200 ml Epichlorhydrin 24 Stunden bei Raumtemperatur quellen. Anschließend wurde unter langsamen Rühren die Temperatur auf 113 - 115° erhöht und 4 Stunden gehalten. Nach Abkühlen wurde über eine Nutsche filtriert und das Copolymerisat mehrmals jeweils 1 Stunde in Aceton ausgerührt. Das acetonfeuchte Copolymerisat wurde bis zur Gewichtskonstanz im Vakuumschrank bei 50° getrocknet. Das Epoxidäquivalent betrug 244 (gemessen nach Axen: Acta Chem. Scand. B 29 (1975) Nr. 4).

**Beispiel 5**

Die Polymerisation von Beispiel 4 wurde analog durchgeführt mit der Ausnahme, daß das Verdünnungsmittel ersetzt wurde durch eine Mischung von 80 g 2-Ethylhexanol und 20 g eines Polyglykols aus Äthylenoxid und Propylenoxyd (Gewichtsverhältnis 1 : 1; statistische Verteilung) mit einem Molekulargewicht von ca. 1200, erhalten durch Anlagerung von Äthylenoxyd und Propylenoxyd an Butanol als Starter. ("Polyglykol B 11/50" der HOECHST AG).

Es wurden 76 g eines kalkweißen runden Perlpolymerisats isoliert, dessen Partikelgröße 50 bis 200 µm betrug, bei einer Rührgeschwindigkeit von 460 UpM.

**Beispiel 6**

Die Polymerisation von Beispiel 4 wurde analog durchgeführt mit der Ausnahme, daß das Verdünnungsmittel ersetzt wurde durch eine Mischung von 70 g 2-Ethylhexanol und 30 g eines Polyglykols mit einem Molekulargewicht von ca. 700, erhalten durch Anlagerung von Propylenoxyd an Butanol als Starter ("Polyglykol B 01/20" der HOECHST AG).

Es wurden 82 g eines kalkweißen, runden Perlpolymerisats isoliert, dessen Partikelgröße 50 bis 200 µm betrug bei einer Rührgeschwindigkeit von 460 UpM.

**Beispiel 7**

Die Polymerisation wurde analog durchgeführt mit der Ausnahme, daß das Verdünnungsmittel ersetzt wurde durch eine Mischung von 80 g 2-Ethylenhexanol und 20 g eines Polyglykols aus Äthylenoxyd und Propylenoxid (Gewichtsverhältnis: 4 : 1; statistische Verteilung; Molekulargewicht: ca. 5000), erhalten durch Anlagerung von Äthylenoxyd und Propylenoxyd an Butanol als Starter. ("Polyglykol P 41/300" der HOECHST AG).

Es wurden 68 g eines kalkweißen, runden Perlpolymerisats isoliert, dessen Partikelgröße im Bereich 50 bis 200 µm lag bei einer Rührdrehzahl von 460 UpM.

**Beispiel 8**

Die Polymerisation von Beispiel 4 wurde analog durchgeführt mit der Ausnahme, daß das Verdünnungsmittel ersetzt wurde durch eine Mischung von 80 g 2-Ethylhexanol und 20 g eines Polyätherglykols aus

Polyoxypropylen und Polyoxyäthylen mit 10 Gew.-% Polyoxyäthylen im Gesamtmolekül, wobei die Polyoxyäthylen-Einheiten an beiden Enden der Polyoxypropylen-Kette (Molekulargewicht: ca. 1750) addiert sind ("Pluronic® polyol 61" der BASF, Wyandotte Corp.).

Es wurden 72 g eines kalkweißen, runden Perlpolymerisats isoliert, dessen Partikelgröße im Bereich 50 bis 200 µm lag bei einer Rührdrehzahl von 460 UpM.

**Beispiel 9**

Die Polymerisation von Beispiel 4 wurde analog durchgeführt mit der Ausnahme, daß das Verdünnungsmittel durch eine Mischung von 100 g 2-Ethylhexanol und 100 g Di-n-butylether ersetzt wurde. Es wurden 83 g eines kalkweißen, völlig runden Perlpolymersats isoliert, dessen Partikelgröße 70 µm betrug, bei einer Rührgeschwindigkeit wie in Beispiel 4.

Das Schüttvolumen des Produkts betrug 2,81 ml/g, im Tetrahydrofuran hatte es das Gelbettvolumen 7,48 ml/g und ein Ausschlußmolekulargewicht für Polystyrol von 2 x $10^6$. Das hydrolysierte Produkt hatte ein Schüttvolumen von 1,6 ml/g, das Gelbettvolumen in Wasser betrug 12,8 ml/g und das Ausschlußmolekulargewicht für Polyethylenglykol war 2 x $10^6$.

**Beispiel 10**

Die Polymerisation von Beispiel 4 wurde analog durchgeführt mit der Ausnahme, daß die dispergierte Phase aus 70 g Vinylacetat, 30 g Divinylethylenharstoff, 1 g Azoisobutyronitril und 158 g Di-n-butylether bestand. Es wurden 77 g eines weißen Perlpolymerisats mit einem mittleren Durchmesser von 200 µm erhalten.

Das Schüttvolumen betrug 2,9 ml/g, das Gelbettvolumen in Tetrahydrofuran lag bei 7,45 ml/g. Gelchromatographisch ließ sich ein Ausschlußmolekulargewicht des Produktes nicht bestimmen: Polystyrol mit M= 25.000.000 wurde mit fast dem Innenvolumen eluiert. Rasterelektronmikroskopische Aufnahmen zeigten Poren von mehr als 100.000 Å Durchmesser.

Das hydrolysierte Produkt hatte ein Schüttvolumen von 6,5 ml/g, was zeigte, daß die Gerüststruktur vollkommen erhalten geblieben war. Das Gelbettvolumen in Wasser betrug 14,5 ml/g; in der gelchromatographischen Untersuchung stand Polyethylenglykol mit dem Molekulargewicht 3,8 x $10^6$ fast das gesamte Innenvolumen zur Verfügung. 10 g des hydrolysierten Copolymerisates wurden nach 24-stündigem Quellen in 100 g Epichlorhydrin langsam unter Rühren auf 110° erwärmt und über 12 Stunden bei dieser Temperatur gehalten. Nach Abkühlen auf Raumtemperatur wurde abgenutscht und das Copolymerisat mehrmals jeweils 2 Stunden langsam in Aceton ausgerührt. Die Trocknung erfolgte im Vakuumschrank bei 50°C. Das Epoxidäquivalent betrug 105 µmol/g Trägersubstanz.

Umsetzung der erfindungsgemäßen perlförmigen Polymerträger mit biologisch aktiven Substanzen

**Beispiel 11**

Zu 100 mg eines nach Beispiel 4 hergestellten Trägers wurden 800 µl einer Trypsinlösung (6,25 mg/ml, 345 U/ml) zugesetzt. Zur Einstellung der Enzymlösung auf pH 7,8 wurde 1-molarer Kaliumphosphatpuffer, zur Stabilisierung des aktiven Zentrums des Enzyms 1,6.$10^{-2}$-molare Benzamidinlösung zugegeben. Die Dauer der Fixierung des Enzyms an den Träger betrug 72 Stunden bei 25°C. Anschließend wurde das nicht kovalent an den Träger gebundene Trypsin über eine Glasfritte abgesaugt und der Rückstand mehrmals mit 1-molarer Natriumchloridlösung, dann mit Pufferlösung ausgewaschen. Die Ausbeute an nutschenfeuchtem Material lag bei 324 mg. Die Messung wurde mit dem Autotitrator bei 37°C und einem pH-Wert von 7,8 mit N'-Benzoyl-L-argininethylesterhydrochlorid (BAEE) durchgeführt und ergab einen Wert von 227,5 U/g im Feuchtzustand oder 356 U/g, bezogen auf das Trockengewicht. Die Blanzierung von Ausgangs- und Waschwasseraktivität ergab eine Fixierungsausbeute von 20 %.

**Beispiel 12**

Zu 200 mg eines nach Beispiel 10 hergestellten epoxidierten Trägers wurden 1500 µl einer Urease-Lösung (30 mg/ml, 51 U/ml), die mit 1-molarem Kaliumphosphatpuffer auf einen pH-Wert von 8,0 eingestellt wurde, gegeben. Nach einer Fixierungsdauer von 16 Stunden bei Raumtemperatur wurde der Träger mit 1-molarer Natriumchloridlösung, anschließend mit Pufferlösung mehrmals gewaschen. Die Ausbeute an nutschenfeuchtem Träger betrug 754 mg. Die Messung mit dem Autotitrator bei 30° und bei pH 8,0, mit

Harnstoff als Substrat, ergab eine Aktivität von 100 U/g (feucht) oder 377 U/g, bezogen auf das Trockengewicht des Trägers. Die Bilanzierung von Ausgangs- und Waschwasseraktivität ergab eine Fixierungsausbeute von 98 %.

**Patentansprüche** für die Vertragsstaaten: BE, CH, DE, FR, GB, IT, LI, LU, NL, SE

1. Vernetztes, perlförmiges Polymerisat, das im wesentlichen aus Vinylacylat-Einheiten und Einheiten eines Vernetzungsmittels besteht, dadurch gekennzeichnet, daß das Vernetzungsmittel die allgemeinen Formeln

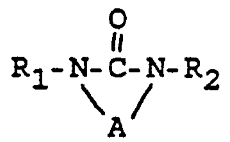

(I)

und/oder

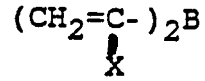

(II)

besitzt, wobei $R_1$, $R_2$ in Formel (I) gleich oder verschieden sein können und Vinyl-, 1-Acyloxy-Vinyl, Allyl-oder 2-Acyloxy-Allyl- bedeuten, A einen zweiwertigen Kohlenwasserstoffrest mit 2 bis 8 C-Atomen darstellt, B in Formel (II) für einen zweiwertigen Kohlenwasserstoffrest mit 1 bis 8 C-Atomen steht, und X Acyloxy bedeutet, wobei die Menge an Vernetzungsmittel-Einheiten bis 1 bis 60 Gew.-%, bezogen auf das Polymere, beträgt, wobei die Acyloxy-Gruppen der Vinylacylat-Einheiten als solche vorliegen oder teilweise oder vollständig durch Hydroxylgruppen ersetzt sind, und wobei die mittlere Teilchengröße der Perlen 20 bis 800 μm und der mittlere Porendurchmesser 2 bis 10000 nm beträgt.

2. Vernetztes Polymerisat nach Anspruch 1, dadurch gekennzeichnet, daß es Vinylacetet-Einheiten enthält.

3. Vernetztes Polymerisat nach Anspruch 1 oder 2, dadurch gekennzeichnet, daß die Acyloxy-Gruppe in den Resten $R_1$, $R_2$ der Formel (I) oder der Acyloxyrest in Formel (II) 2 bis 6 C-Atome besitzt.

4. Vernetztes Polymerisat nach einem oder mehreren der Ansprüche 1 bis 3, dadurch gekennzeichnet, daß die Reste $R_1/R_2$ der Formel (I) jeweils für Vinyl- stehen.

5. Vernetztes Polymerisat nach einem oder mehreren der Anspruche 1 bis 4, dadurch gekennzeichnet, daß die Menge an Vernetzungsmittel-Einheiten 1 bis 50 Gew.-% beträgt.

6. Vernetztes Polymerisat nach einem oder mehreren der Ansprüche 1 bis 5, dadurch gekennzeichnet, daß die Menge an Vernetzungsmittel-Einheiten 1 bis 40 Gew.-% beträgt.

7. Vernetztes Polymerisat nach einem oder mehreren der Ansprüche 1 bis 6, dadurch gekennzeichnet, daß zumindest 10 Gew.-% der Acyloxy-Gruppen der Vinylacylat-Einheiten durch Hydroxylgruppen ersetzt sind.

8. Verfahren zur Herstellung des vernetzten Polymerisates gemäß Anspruch 1 bis 7 durch Copolymerisation von Vinylacylat mit einem Vernetzungsmittel in Gegenwart eines Dispersionsmittels und gegebenenfalls teilweise oder vollständige Verseifung des erhaltenen Polymerisates, dadurch gekennzeichnet, daß das Vernetzungsmittel die Formeln (I) und/oder (II) gemäß Anspruch 1 besitzt.

9. Verfahren nach Anspruch 8, dadurch gekennzeichnet, daß das Vinylacylat Vinylacetat darstellt.

10. Vefahren nach Anspruch 8 oder 9, dadurch gekennzeichnet, daß noch ein weiteres mit dem Vinylacylat copolymerisierbares Monomeres zugegen ist.

11. Verfahren nach einem oder mehreren der Ansprüche 8 bis 10, dadurch gekennzeichnet, daß die Polymerisation als Perlpolymerisation durchgeführt.

12. Verfahren nach Anspruch 11, dadurch gekennzeichnet, daß als Dispersionsmittel eine alkalische wäßrige Pufferlösung verwendet wird.

13. Verfahren nach Anspruch 12, dadurch gekennzeichnet, das das Dispersionsmittel 0 -50 Gew.-% eines Elektrolyten enthält.

14. Verfahren nach einem oder mehreren der Ansprüche 11 bis 13, dadurch gekennzeichnet, daß das Dispersionsmittel eine nicht-ionogene grenzflächenaktive Verbindung als Dispersionsstabilisator enthält.

15. Verfahren nach einem oder mehreren der Ansprüche 8 bis 14, dadurch gekennzeichnet, daß die Polymerisation in Gegenwart eines Dialkylethers mit mindestens 6 C-Atomen als inertes Verdünnungsmittel durchgeführt wird.

16. Verfahren zur Herstellung des vernetzten Polymerisates gemäß Anspruch 7, dadurch gekennzeichnet, daß das vinylacylgruppenhaltige Polymere einer Verseifung in der Weise unterworfen wurde, daß mehr als 70 % der Acylatgruppen durch OH-Gruppen ersetzt sind.

17. Verwendung des vernetzten Polymerisats nach einem oder mehreren der Ansprüche 1 bis 7 als

Adsorbens in der Chromatographie.

18. Verwendung des vernetzten Polymerisates nach einem oder mehreren der Ansprüche 1 bis 7, gegebenenfalls nach Umsatz mit Spacern, zur Herstellung trägergebundener, biologisch aktiver Substanzen.

19. Verwendung nach Anspruch 18, dadurch gekennzeichnet, daß der Spacer Epichlorhydrin darstellt.

**Patentansprüche** für den Vertragsstaat AT

1. Verfahren zur Herstellung eines vernetzten, perlförmigen Polymerisates durch Copolymerisation von Vinylacylat mit einem Vernetzungsmittel sowie gegebenenfalls einem weiteren mit dem Vinylacylat copolymerisierbaren Monomeren in Gegenwart eines Dispersionsmittels und gegebenenfalls teilweise oder vollständige Verseifung des erhaltenen Polymerisates, dadurch gekennzeichnet, daß das Vernetzungsmittel die allgemeine Formeln

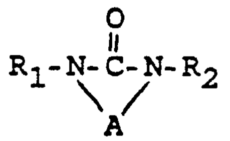

(I)

und/oder

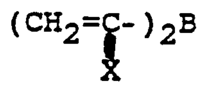

(II)

besitzt, wobei $R_1$, $R_2$ in Formel (I) gleich oder verschieden sein können und Vinyl-, 1-Acyloxy-Vinyl, Allyl- oder 2-Acyloxy-Allyl- bedeuten, A einen zweiwertigen Kohlenwasserstoffrest mit 2 bis 8 C-Atomen darstellt, B in Formel (II) für einen zweiwertigen Kohlenwasserstoffrest mit 1 bis 8 C-Atomen steht, X Acyloxy bedeutet und die erhaltenen Perlen eine mittlere Teilchengröße von 10 bis 800 µm und einen mittleren Porendurchmesser von 2 bis 10000 nm aufweisen.

2. Verfahren nach Anspruch 1, dadurch gekennzeichnet, daß die Acyloxy-Gruppe in den Resten $R_1$, $R_2$ der Formel (I) oder der Acyloxyrest in Formel (II) 2 bis 6 C-Atome besitzt.

3. Verfahren nach Anspruch 1 und/einer 2, dadurch gekennzeichnet, daß die Reste $R_1/R_2$ der Formel (I) jeweils für Vinyl stehen.

4. Verfahren nach einem oder mehreren der Ansprüche 1 bis 3, dadurch gekennzeichnet, daß das Vernetzungsmittel in einer Menge eingesetzt wird, daß davon im Polymerisat 1 bis 50 Gew.-%, bezogen auf das Polymerisat, vorhanden sind.

5. Verfahren nach einem oder mehreren der Ansprüche 1 bis 4, dadurch gekennzeichnet, daß das Vinylacylat Vinylacetat darstellt.

6. Verfahren nach einem oder mehreren der Ansprüche 1 bis 5, dadurch gekennzeichnet, daß die Polymerisation als Perlpolymerisation durchgeführt wird.

7. Verfahren nach Anspruch 6, dadurch gekennzeichnet, daß als Dispersionsmittel eine alkalische wäßrige Pufferlösung verwendet wird.

8. Verfahren nach Anspruch 7, dadurch gekennzeichnet, daß das Dispersionsmittel 0 - 50 Gew.-% eines Elektrolyten enthält.

9. Verfahren nach einem oder mehreren der Ansprüche 6 bis 8, dadurch gekennzeichnet, daß das Dispersionsmittel eine nicht-ionogene grenzflächenaktive Verbindung als Dispersionsstabilisator enthält.

10. Verfahren nach einem oder mehreren der Ansprüche 1 bis 9, dadurch gekennzeichnet, daß die Polymerisation in Gegenwart eines Dialkylethers mit mindestens 6 C-Atomen als inertes Verdünnungsmittel durchgeführt wird.

11. Verfahren nach einem oder mehreren der Ansprüche 1 bis, 10, dadurch gekennzeichnet, daß das vinylacylgruppenhaltige Polymere einer Verseifung in der Weise unterworfen wurde, daß mehr als 70 % der Acylatgruppen durch OH-Gruppen ersetzt sind.

12. Verwendung des vernetzten Polymerisats, erhalten nach einem oder mehreren der Ansprüche 1 bis 11 als Adsorbens in der Chromatographie.

13. Verwendung des vernetzten Polymerisates, erhalten nach einem oder mehreren der Ansprüche 1 bis 11, gegebenenfalls nach Umsatz mit Spacern, zur Herstellung trägergebundener, biologisch aktiver Substanzen.

14. Verwendung nach Anspruch 13, dadurch gekennzeichnet, daß der Spacer Epichlorhydrin darstellt.

**Claims** for the contracting states: BE, CH, DE, FR, GB, IT, LI, LU, NL, SE

1. A crosslinked polymer in the form of beads which substantielly comprises vinyl acylate units and units of a crosslinking agent which has the formulae

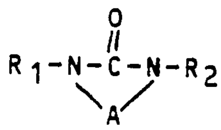

(I)

and/or

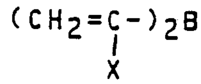

(II)

where $R_1$ and $R_2$ in the formula (I) can be identical or different and each denotes vinyl-, 1-acyloxyvinyl, allyl- or 2-acyloxyallyl, A represents a divalent hydrocarbon radical of 2 to 8 carbon atoms, B in the formula (II) stands for a divalent hydrocarbon radical of 1 to 8 carbon atoms and X denotes acyloxy, the amount of these crosslinking agent units accounting for 1 to 60 % by weight of the polymer, the acyloxy groups of the vinyl acylate units being present as such or having been partially or completely replaced by hydroxyl groups, and the average particle size of the beads being 20 to 800 µm and the mean pore diameter being 2 to 10,000 nm.

2. The crosslinked polymer as claimed in claim 1, containing vinyl acetate units.

3. The crosslinked polymer as claimed in claim 1 or 2, wherein the acyloxy group in the radicals $R_1$ and $R_2$ of the formula (I) or the acyloxy radical in the formula (II) has 2 to 6 carbon atoms.

4. The crosslinked polymer as claimed in one or more of claims 1 to 3, wherein the radicals $R_1/R_2$ of the formula (I) each stand for vinyl.

5. The crosslinked polymer as claimed in one or more of claims 1 to 4, wherein the amount of crosslinking agent units is 1 to 50 % by weight.

6. The crosslinked polymer as claimed in one or more of claims 1 to 5, wherein the amount of crosslinking agent units is 1 to 40 % by weight.

7. The crosslinked polymer as claimed in one or more of claims 1 to 6, wherein at least 10 % by weight of the acyloxy groups of the vinyl acylate units are replaced by hydroxyl groups.

8. A process for preparing the crosslinked polymer as claimed in claims 1 to 7, by copolymerizing vinyl acylate with a crosslinking agent in the presence of a dispersion medium and if desired partially or completely hydrolyzing the resulting polymer, wherein the crosslinking agent has the formulae (I) and/or (II) of claim 1.

9. The process as claimed in claim 8, wherein the vinyl acylate is vinyl acetate.

10. The process as claimed in claim 8 or 9, wherein an additional monomer copolymerizable with the vinyl acylate is also present.

11. The process as claimed in one or more of claims 8 to 10, wherein the polymerization is carried out as a bead polymerization.

12. The process as claimed in claim 11, wherein the dispersion medium is an alkaline aqueous buffer solution.

13. The process as claimed in claim 12, wherein the dispersion medium contains 0 - 50 % by weight of an electrolyte.

14. The process as claimed in one or more of claims 11 to 13, wherein dispersion medium contains a nonionic surfactant as dispersion stabilizer.

15. The process as claimed in one or more of claims 8 to 14, wherein the polymerization is carried out in the presence of a dialkyl ether of at least 6 carbon atoms as inert diluent.

16. A process for preparing the crosslinked polymer as claimed in claim 7, wherein the polymer containing vinyl acyl groups has been subjected to such a hydrolysis that more than 70 % of the acylate groups have been replaced by OH groups.

17. Use of the crosslinked polymers as claimed in one or more of claims 1 to 7 as an adsorbent in chromatography.

18. Use of the crosslinked polymer as claimed in one or more of claims 1 to 7, if desired after conversion with spacers, for preparing carrier-bounded biologically active substances.

19. The use as claimed in claim 18, wherein the spacer is epichlorohydrin.

**Claims** for the contracting state AT

1. A process for preparing a crosslinked polymer in the form of beads by copolymerizing vinyl acylate with a crosslinking agent and, if appropriate, a further monomer copolymerizable with the vinyl acylate in ther presence of a dispersion medium and if desired partially or completely hydrolyzing the resulting polymer, wherein the crosslinking agent has the formulae

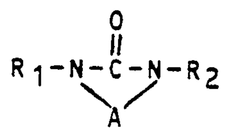 (I)

and/or

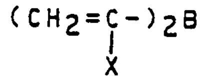 (II)

where $R_1$ and $R_2$ in the formula (I) can be identical or different and each denotes vinyl, 1-acyloxyvinyl, allyl or 2-acyloxyallyl, A represents a divalent hydrocarbon radical of 2 to 8 carbon atoms, B in the formula (II) stands for a divalent hydrocarbon radical of 1 to 8 carbon atoms and X denotes acyloxy, and the beads obtained have an average particle size of 10 to 800 µm and an average pore diameter of 2 to 10,000 nm.

2. The process as claimed in claim 1, wherein the acyloxy group in the radicals $R_1$ and $R_2$ of the formula (I) or the acyloxy radical in the formula (II) has 2 to 6 carbon atoms.

3. The process as claimed in claim 1 and/or 2, wherein the radicals $R_1/R_2$ of the formula (I) each stand for vinyl.

4. The process as claimed in one or more of claims 1 to 3, wherein the crosslinking agent is employed in such an amount that the polymer contains 1 to 50 % by weight, of it, based on the polymer.

5. The process as claimed in one or more of claims 1 to 4, wherein the vinyl acylate is vinyl acetate.

6. The process as claimed in one or more of claims 1 to 5, wherein the polymerization is carried out as a bead polymerization.

7. The process as claimed in claim 6, wherein the dispersion medium is an alkaline aqueous buffer solution.

8. The process as claimed in claim 7, wherein the dispersion medium contains 0 - 50 % by weight of an electrolyte.

9. The process as claimed in one or more of claims 6 to 8, wherein the dispersion medium contains a nonionic surfactant as dispersion stabilizer.

10. The process as claimed in one or more of claims 1 to 9, wherein the polymerization is carried out in the presence of a dialkyl ether of at least 6 carbon atoms as inert diluent.

11. The process as claimed in one or more of claims 1 to 10, wherein the polymer containing vinyl acyl groups has been subjected to such a hydrolysis that more than 70 % of the acylate groups have been replaced by OH groups.

12. Use of the crosslinked polymer obtained as claimed in one or more of claims 1 to 11 as an adsorbent in chromatography.

13. Use of the crosslinked polymer obtained as claimed in one or more of claims 1 to 11, if desired after conversion with spacers, for preparing carrier-supported biologically active substances.

14. The use as claimed in claim 13, wherein the spacer is epichlorohydrin.

**Revendications** pour les Etats contractants BE CH DE FR GB IT LI LU NL SE

1. Polymère en perles, réticulé, constitué pour substan tiel d'unités acylate de vinyle et d'unités d'un agent de réticulation, caractérisé en ce que l'agent de réticulation a les formules générales

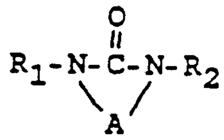

(I)

et/ou

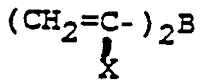

(II)

où $R_1$ et $R_2$, dans la formule (I), peuvent être identiques ou différents et représentent les radicaux vinyle, acyloxy-1 vinyle, allyle ou acyloxy-2 allyle; A est un radical hydrocarboné divalent ayant de 2 à 8 atomes de carbone; B, dans la formule (II), est un radical hydrocarboné divalent ayant de 1 à 8 atomes de carbone; et X est un radical acyloxy; la quantité d'unités "agent de réticulation" étant comprise entre 1 et 60 % en poids par rapport au polymère, les groupes acyloxy des unités acylate de vinyle se présentant en tant que tels ou étant remplacés, en totalité ou en partie, par des groupes hydroxyle, la granulométrie moyenne des perles étant de 200 à 800 µm et le diamètre moyen des pores étant de 2 à 10 000 nm.

2. Polymère réticulé selon la revendication 1, caractérisé en ce qu'il comporte des unités acétate de vinyle.

3. Polymère réticulé selon la revendication 1 ou 2, caractérisé en ce que le groupe acyloxy des radicaux $R_1$, $R_2$ de la formule (I), ou le radical acyloxy de la formule (II), ont de 2 à 6 atomes de carbone.

4. Polymère réticulé selon l'une ou plusieurs des revendications 1 à 3, caractérisé en ce que des radicaux $R_1$ et $R_2$, dans la formule (I), est le radical vinyle.

5. Polymère réticulé selon l'une ou plusieurs des revendications 1 à 4, caractérisé en ce que la quantité d'unités "agent de réticulation" est de 1 à 50 % en poids.

6. Polymère réticulé selon l'une ou plusieurs des revendications 1 à 5, caractérisé en ce que la quantité d'unités "agent de réticulation" est de 1 à 40 % en poids.

7. Polymère réticulé selon l'une ou plusieurs des revendications 1 à 6, caractérisé en ce qu'au moins 10 % en poids des groupes acyloxy des unités acylate de vinyle sont remplacés par des groupes hydroxyle.

8. Procédé de préparation du polymère réticulé selon les revendications 1 à 7, par copolymérisation d'acylate de vinyle avec un agent de réticulation, en présence d'un dispersant, et éventuellement saponification, partielle ou totale, du polymère obtenu, caractérisé en ce que l'agent de réticulation a les formules (I) et/ou (II) selon la revendication 1.

9. Procédé selon la revendication 8, caractérisé en ce que l'acylate de vinyle est l'acétate de vinyle.

10. Procédé selon la revendication 8 ou 9, caractérisé en ce qu'un autre monomère, copolymérisable avec l'acylate de vinyle, est encore présent.

11. Procédé selon l'une ou plusieurs des revendications 8 à 10, caractérisé en ce que la polymérisation est réalisée sous la forme d'une polymérisation en perles.

12. Procédé selon la revendication 11, caractérisé en ce qu'on utilise comme dispersant une solution tampon aqueuse alcaline.

13. Procédé selon la revendication 12, caractérisé en ce que le dispersant contient de 0 à 50 % en poids d'un électrolyte.

14. Procédé selon l'une ou plusieurs des revendications 11 à 13, caractérisé en ce que le dispersant contient comme stabilisant de dispersion un composé surfactif non-ionique.

15. Procédé selon l'une ou plusieurs des revendications 8 à 14, caractérisé en ce que la polymérisation est mise en oeuvre en présence, en tant que diluant inerte, d'un dialkyléther comportant au moins 6 atomes de carbone.

16. Procédé pour la préparation du polymère réticulé selon la revendication 7, caractérisé en ce que le polymère contenant des groupes vinylacyle a été soumis à une saponification de façon que plus de 70 % des groupes acylate soient remplacés par des groupes OH.

17. Utilisation du polymère réticulé selon l'une ou plusieurs des revendications 1 à 7 en tant qu'adsorbant en chromatographie.

18. Utilisation du polymère réticulé selon l'une ou plusieurs des revendications 1 à 7, éventuellement après réaction sur des composé intercalaires (Spacers) pour préparer des substances biologiquement actives, fixées à un support.

19. Utilisation selon la revendication 18, carctérisé en ce que le composé intercalaire est l'épichlorhydrine.

**Revendications** pour l'Etat contractant AT

1. Procédé pour la préparation d'un polymère en perles, réticulé, par copolymérisation d'un acylate de vinyle avec un agent de réticulation, et éventuellement avec un monomère supplémentaire copolymérisable avec l'acylate de vinyle, en présence d'un dispersant, et éventuellement saponification, partielle ou totale, du polymère obtenu, caractérisé en ce que l'agent de réticulation a les formules générales suivantes:

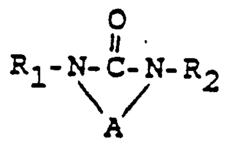

(I)

et/ou

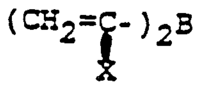

(II)

où $R_1$ et $R_2$, dans la formule (I), peuvent être identiques ou différents, et représentent chacun les radicaux vinyle, acyloxy-1 vinyle, allyle ou acyloxy-2 allyle; A est un radical hydrocarboné divalent ayant de 2 à 8 atomes de carbone; B, dans la formule (II), est un radical hydrocarboné divalent ayant de 1 à 8 atomes de carbone; X est un radical acyloxy; et les perles obtenues ont une granulométrie moyenne de 10 à 800 μm et un diamètre moyen des pores de 2 à 10 000 nm.*

2. Procédé selon la revendication 1, caractérisé en ce que le groupe acyloxy, dans les radicaux $R_1$ et $R_2$ de la formule (I), ou le radical acyloxy de la formule (II), a de 2 à 6 atomes de carbone.

3. Procédé selon la revendication 1 et/ou 2, caractérisé en ce que les radicaux $R_1$ et $R_2$, dans la formule (I), sont chacun le radical vinyle.

4. Procédé selon l'une ou plusieurs des revendications 1 à 3, caractérisé en ce que l'agent de réticulation est utilisé en une quantité correspondant dans le polymère à 1 à 50 % en poids par rapport au polymère.

5. Procédé selon l'une ou plusieurs des revendications 1 à 4, caractérisé en ce que l'acylate de vinyle est l'acétate de vinyle.

6. Procédé selon l'une ou plusieurs des revendications 1 à 5, caractérisé en ce que la polymérisation est mise en oeuvre sous la forme d'une polymérisation en perles.

7. Procédé selon la revendication 6, caractérisé en ce qu'on utilise comme dispersant une solution tampon aqueuse alcaline.

8. Procédé selon la revendication 7, caractérisé en ce que le dispersant contient de 0 à 50 % en poids d'un électrolyte.

9. Procédé selon l'une ou plusieurs des revendications 6 à 8, caractérisé en ce que le dispersant contient comme stabilisant de dispersion un composé surfactif non-ionique.

10. Procédé selon l'une ou plusieurs des revendications 1 à 9, caractérisé en ce que la polymérisation est mise en oeuvre en présence, en tant que diluant inerte, d'un dialkyléther ayant au moins 6 atomes de carbone.

11. Procédé selon l'une ou plusieurs des revendications 1 à 10, caractérisé en ce que le polymère comprenant des groupes vinylacyle a été soumis à une saponification conduisant au remplacement de plus de 70 % des groupes acylate par des groupes OH.

12. Utilisation du polymère réticulé, obtenu selon l'une ou plusieurs des revendications 1 à 11, comme adsorbant en chromatographie.

13. Utilisation du polymère réticulé obtenu selon l'une ou plusieurs des revendications 1 à 11, éventuellement après réaction sur des composés intercalaires (spacers) pour préparer des substances biologiquement actives fixées au support.

14. Utilisation selon la revendication 13, caractérisée en ce que le composé intercalaire est l'épichlorhydrine.